# EUROPEAN PATENT APPLICATION

(11) **EP 3 679 921 A1**
(43) Date of publication of application: **15.07.2020**
(21) Application number: 18854317.7
(22) Date of filing: 28.08.2018
(51) Int. Cl.: A61K 8/87, A61K 8/41, A61K 8/42, A61Q 5/00

(54) **HAIR COSMETIC COMPOSITION CONTAINING AQUEOUS GELLING AGENT, AND METHOD FOR PRODUCING AQUEOUS GELLING AGENT AND HAIR COSMETIC COMPOSITION**

(30) Priority: 07.09.2017 JP 2017172299
(71) Applicant: Adeka Corporation, Tokyo 116-8554 (JP)
(72) Inventor: SAKAMOTO, Takao, Tokyo 116-8554 (JP); TSUSHIMA, Yasuhiro, Tokyo 116-8554 (JP); TAKEISHI, Yuki, Tokyo 116-8554 (JP)
(74) Representative: Kador & Partner PartG mbB
(86) International application number: PCT/JP2018/031704
(87) International publication number: WO 2019/049722

(57) **Abstract**

The present invention has an object to provide a hair cosmetic composition which exhibits flow properties suitable for a hair cosmetic, has high temperature stability in terms of the viscosity thereof, and imparts a favorable texture to hair when applied thereto. In order to achieve such an object, provided is a hair cosmetic composition including a component (A), which is an aqueous gelling agent represented by the general formula (1) below, and component (B), which includes either one of or both of a cationic surfactant and a cationic polymer, wherein the component (A) presents a viscosity of an aqueous solution containing 1 mass% of at 25°C of 1,000 to 5,000 mPa·s and a clouding point of the aqueous solution containing 1 mass% of component (A) of 60°C to 80°C, and has a weight average molecular weight of 10,000 to 30,000. Also provided are a method for producing the aqueous gelling agent and a method for producing the hair cosmetic composition. 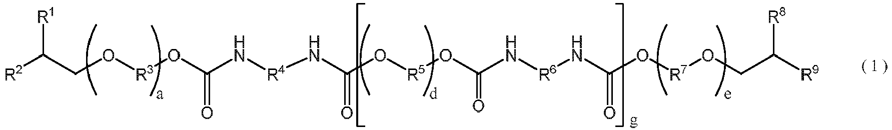 In the formula, R¹, R², R⁸ and R⁹ each independently denote a hydrocarbon group having 4 to 20 carbon atoms, R³, R⁵ and R⁷ each denote represent a divalent hydrocarbon group having 2 to 4 carbon atoms, R⁴ and R⁶ each independently denote a divalent hydrocarbon group having 3 to 16 carbon atoms, a and e each independently denote a number from 10 to 100, d denotes a number from 100 to 500, and g denotes a number from 0 to 10.

## Description

### Technical Field

This invention relates to a hair cosmetic composition which exhibits flow properties suitable for a hair cosmetic, has high temperature stability in terms of the viscosity thereof, and imparts a favorable texture to hair when applied thereto; a method for producing an aqueous gelling agent contained in the hair cosmetic composition; and a method for producing the hair cosmetic composition.

### Background Art

Natural gelling agents, such as carboxymethyl cellulose and hydroxyethyl cellulose, alkali thickening type gelling agents that are thickened by an alkali, such as poly(acrylic acid) and poly(acrylic acid)-containing copolymers, and urethane type gelling agents, such as urethane-modified polyethers, and the like, are known as gelling agents able to be used in cosmetics. Of these, urethane type gelling agents can allow a variety of products to be gelated more freely than other gelling agents, and can impart a wide variety of viscosities to products to which such gelling agents are added, and many types of such gelling agents are produced and frequently used for reasons such as hardly being affected by pH or salts.

Of these, hydrophobically-modified polyether urethanes form elastic gels having a characteristic gelatinous texture and can give gels having excellent temperature stability, and are therefore widely blended and used in cosmetics used in a variety of applications (for example, see Patent Documents 1 to 4).

Furthermore, hydrophobically modified polyether urethanes are hardly affected by pH or salts, and are often advantageously used in hair cosmetics in which large amounts of cationic surfactants are blended. For example, Patent Document 5 discloses a hair treatment composition characterized by containing a hydrophobically modified polyether urethane as a component (A) and a cationic surfactant as a component (B), and Patent Document 6 discloses a hair cosmetic that contains a di-long-chain type cationic activating agent as a component (A) at a quantity of 0.01 to 10 mass% relative to the overall quantity of the hair cosmetic, a hydrophobically modified polyether urethane as a component (B) at a quantity of 0.01 to 10 mass% relative to the overall quantity of the hair cosmetic, and a higher alcohol as a component (C) at a quantity of 0.01 to 30 mass% relative to the overall quantity of the hair cosmetic.

### Citation List

### Patent Document

[Patent Document 1] Japanese Patent Application Publication No. 2002-105493
[Patent Document 2] Japanese Patent Application Publication No. 2011-006371
[Patent Document 3] Japanese Patent Application Publication No. 2016-023180
[Patent Document 4] Japanese Patent Application Publication No. 2014-040385
[Patent Document 5] Japanese Patent Application Publication No. 2002-080329
[Patent Document 6] Japanese Patent Application Publication No. 2006-225318

### Summary of Invention

### Technical Problem

Performance required of a hair cosmetic varies according to the mode of use of the hair cosmetic. However, examples of primary properties required of hair cosmetics include exhibiting flow properties suitable for a hair cosmetic, having high temperature stability in terms of the viscosity thereof, and imparting a favorable texture to hair when applied thereto. Even if the hair cosmetics disclosed in Patent Documents 5 and 6 satisfy some of these properties, these hair cosmetics cannot satisfy all of these properties to a sufficiently practicable level, and are unsatisfactory in terms of these properties.

### Solution to Problem

As a result of intensive studies, the inventors of this invention have found a hair cosmetic composition which exhibits flow properties suitable for a hair cosmetic, has high temperature stability in terms of the viscosity thereof and imparts a favorable texture to hair when applied thereto in comparison with hair cosmetics known in the past, and thereby completed this invention. That is, this invention is a hair cosmetic composition comprising components (A) and (B) below, wherein the component (A) presents a viscosity of an aqueous solution containing 1 mass% of at 25°C of 1,000 to 5,000 mPa·s and a clouding point of the aqueous solution containing 1 mass% of component (A) of 60°C to 80°C, and has a weight average molecular weight of 10,000 to 30,000; component (A): an aqueous gelling agent represented by general formula (1) below.

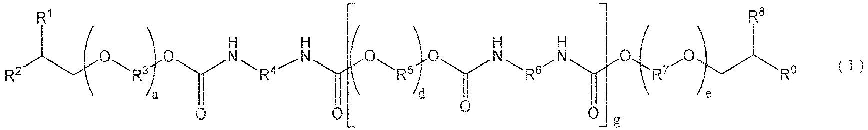

In the formula, R¹, R², R⁸ and R⁹ each independently denote a hydrocarbon group having 4 to 20 carbon atoms, R³, R⁵ and R⁷ each independently denote a divalent hydrocarbon group having 2 to 4 carbon atoms, R⁴ and R⁶ each independently denote a divalent hydrocarbon group having 3 to 16 carbon atoms, a and e each independently denote a number from 10 to 100, d denotes a number from 100 to 500, and g denotes a number from 0 to 10; and
component (B): either one of or both of a cationic surfactant and a cationic polymer

In addition, this invention provides a method for producing an aqueous gelling agent represented by general formula (1), in which compounds represented by general formulae (2) to (4) and higher fatty acid metal salts as catalysts are used, described later.

Furthermore, this invention provides a method for producing a hair cosmetic composition, the method comprising a step of combining the aqueous gelling agent, which has been obtained using the production method mentioned above, with either one of or both of a cationic surfactant and a cationic polymer.

### Advantageous Effects of Invention

Compared to conventional hair cosmetics, this invention can provide a hair cosmetic composition which exhibits flow properties suitable for a hair cosmetic, has high temperature stability in terms of the viscosity thereof, and imparts a favorable texture to hair when applied thereto.

### Description of Embodiments

Component (A) that constitutes the hair cosmetic composition of this invention is an aqueous gelling agent represented by general formula (1) below.

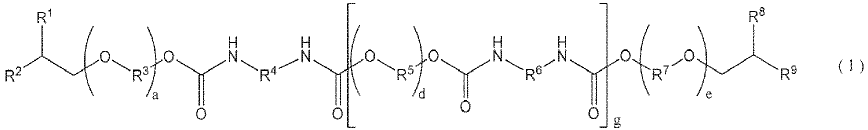

In the formula, R¹, R², R⁸ and R⁹ each independently denote a hydrocarbon group having 4 to 20 carbon atoms, R³, R⁵ and R⁷ each denote a divalent hydrocarbon group having 2 to 4 carbon atoms, R⁴ and R⁶ each independently denote a divalent hydrocarbon group having 3 to 16 carbon atoms, a and e each independently denote a number from 10 to 100, d denotes a number from 100 to 500, and g denotes a number from 0 to 10.

In general formula (1), R¹, R², R⁸ and R⁹ each independently denote a hydrocarbon group having 4 to 20 carbon atoms. Examples of such a hydrocarbon group include saturated aliphatic hydrocarbon groups such as an n-butyl group, an isobutyl group, an s-butyl group, a t-butyl group, an n-pentyl group, a branched pentyl group, a secondary pentyl group, a tertiary pentyl group, an n-hexyl group, a branched hexyl group, an secondary hexyl group, a tertiary hexyl group, an n-heptyl group, a branched heptyl group, a secondary heptyl group, a tertiary heptyl group, an n-octyl group, a 2-ethylhexyl group, a branched octyl group, a secondary octyl group, a tertiary octyl group, an n-nonyl group, a branched nonyl group, a secondary nonyl group, a tertiary nonyl group, an n-decyl group, a branched decyl group, a secondary decyl group, a tertiary decyl group, an n-undecyl group, a branched undecyl group, a secondary undecyl group, a tertiary undecyl group, an n-dodecyl group, a branched dodecyl group, a secondary dodecyl group, a tertiary dodecyl group, an n-tridecyl group, a branched tridecyl group, a secondary tridecyl group, a tertiary tridecyl group, an n-tetradecyl group, a branched tetradecyl group, a secondary tetradecyl group, a tertiary tetradecyl group, an n-pentadecyl group, branched pentadecyl group, a secondary pentadecyl group, tertiary pentadecyl group, an n-hexadecyl group, a branched hexadecyl group, a secondary hexadecyl group, a tertiary hexadecyl group, an n-heptadecyl group, a branched heptadecyl group, a secondary heptadecyl group, a tertiary heptadecyl group, an n-octadecyl group, a branched octadecyl group, a secondary octadecyl group, a tertiary octadecyl group, an n-nonadecyl group, a branched nonadecyl group, a secondary nonadecyl group, a tertiary nonadecyl group, an n-eicosyl group, a branched eicosyl group, a secondary eicosyl group, and a tertiary eicosyl group; and unsaturated aliphatic hydrocarbon groups such as a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group, a 1-heptenyl group, a 6-heptenyl group, a 1-octenyl group, a 7-octenyl group, a 8-nonenyl group, a 1-decenyl group, a 9-decenyl group, a 10-undecenyl group, a 1-dodecenyl group, a 4-dodecenyl group, a 11-dodecenyl group, a 12-tridecenyl group, a 13-tetradecenyl group, a 14-pentadecenyl group, a 15-hexadecenyl group, a 16-heptadecenyl group, a 1-octadecenyl group, a 17-octadecenyl group, a 1-nonadecenyl group, and a 1-eicosenyl group;

aromatic hydrocarbon groups such as a phenyl group, a toluyl group, a xylyl group, a cumenyl group, a mesityl group, a benzyl group, a phenethyl group, a styryl group, a cinnamyl group, a benzhydryl group, a trityl group, an ethylphenyl group, a propylphenyl group, a butylphenyl group, a pentylphenyl group, a hexylphenyl group, a heptylphenyl group, an octylphenyl group, a nonylphenyl group, a decylphenyl group, an undecylphenyl group, a dodecylphenyl group, a styrenated phenyl group, a p-cumylphenyl group, a phenylphenyl group, a benzylphenyl group, an α-naphthyl group, and a β-naphthyl group; and alicyclic hydrocarbon groups such as a cyclopropyl group, a cyclobutyl group, a cyclopentyl group, a cyclohexyl group, a cycloheptyl group, a cyclooctyl group, a methylcyclopentyl group, a methylcyclohexyl group, a methylcycloheptyl group, a methylcyclooctyl group, a 4,4,6,6-tetramethylcyclohexyl group, a 1,3-dibutylcyclohexyl group, a norbornyl group, a bicyclo[2.2.2]octyl group, an adamantyl group, a 1-cyclobutenyl group, a 1-cyclopentenyl group, a 3-cyclopentenyl group, a 1-cyclohexenyl group, a 3-cyclohexenyl group, a 3-cycloheptenyl group, a 4-cyclooctenyl group, a 2-methyl-3-cyclohexenyl group, and a 3,4-dimethyl-3-cyclohexenyl group. In this invention, if R¹, R², R⁸ and R⁹ are not such hydrocarbon groups, it is not possible to obtain a hair cosmetic composition that satisfies all of the advantageous effects of this invention, and especially a hair cosmetic composition that imparts a favorable texture to hair when applied thereto.

R¹, R², R⁸ and R⁹ may be the same as, or different from, each other. Of these, saturated aliphatic hydrocarbon groups and unsaturated aliphatic hydrocarbon groups are preferred, saturated aliphatic hydrocarbon groups are more preferred, saturated aliphatic hydrocarbon groups having 5 to 18 carbon atoms are further preferred, saturated aliphatic hydrocarbon groups having 8 to 14 carbon atoms are yet further preferred, and saturated aliphatic hydrocarbon groups having 10 to 12 carbon atoms are most preferred from the perspectives of readily achieving the advantageous effect of this invention, ease of procurement of raw materials and ease of production.

In general formula (1), R³, R⁵ and R⁷ each independently denote a divalent hydrocarbon group having 2 to 4 carbon atoms. Examples of such a hydrocarbon group include an ethylene group; a propane-1,3-diyl (linear propylene) group; branched propylene groups such as a propane-1,2-diyl group and a propane-2,2-diyl group; linear butylene groups such as a butane-1,4-diyl group, a butane-1,2-diyl group, a butane-1,3-diyl group, a butane-2,3-diyl group, a butane-1,1-diyl group, and a butane-2,2-diyl group; and branched butylene groups such as a 2-methylpropane-1,3-diyl group and a 2-methylpropane-1,2-diyl group. Of these, linear divalent hydrocarbon groups having 2 to 4 carbon atoms are preferred, an ethylene group and a propane-1,3-diyl (linear propylene) group are more preferred, and an ethylene group is further preferred from the perspective of readily achieving the advantageous effect of this invention. Moreover, the R³ groups may all be the same as, or different from, each other, the R⁵ groups may all be the same as, or different from, each other, and the R⁷ groups may all be the same as, or different from, each other.

In general formula (1), R⁴ and R⁶ each independently denote a divalent hydrocarbon group having 3 to 16 carbon atoms. Examples of such hydrocarbon groups include divalent aliphatic hydrocarbon groups having 3 to 16 carbon atoms, divalent aromatic hydrocarbon groups having 3 to 16 carbon atoms and divalent alicyclic hydrocarbon groups having 3 to 16 carbon atoms. These hydrocarbon groups may be any type as long as the number of carbon atoms falls within the range 3 to 16, but a group obtained by removing two isocyanate groups from a diisocyanate compound represented by general formula (4), which is described below, is preferred from the perspectives of ease of production and ease of procurement of raw materials. A detailed explanation of this is given later.

a and e each independently denote a number from 10 to 100. Within this range, these values are preferably 12 to 50, and more preferably 15 to 30, from the perspectives of ease of production and procurement of raw materials and readily achieving the advantageous effect of this invention.

d denotes a number from 100 to 500. Within this range, the value of d is preferably 120 to 450, more preferably 150 to 400, further preferably 180 to 350, and most preferably 200 to 300, from the perspective of readily achieving the advantageous effect of this invention.

g denotes a number from 0 to 10. Within this range, the value of g is preferably 0 to 8, and more preferably 0 to 6, from the perspective of readily achieving the advantageous effect of this invention. Moreover, an aqueous gelling agent in which the value of g is 0 behaves like a gelling accelerator when used in combination with an aqueous gelling agent in which the value of g is 1 to 10. Therefore, from the perspective of more readily achieving the advantageous effect of this invention, a mixture of an aqueous gelling agent in which the value of g is 0 and an aqueous gelling agent in which the value of g is 1 to 10 is more preferred, a mixture of an aqueous gelling agent in which the value of g is 0 and an aqueous gelling agent in which the value of g is 1 to 8 is further preferred, and a mixture of an aqueous gelling agent in which the value of g is 0 and an aqueous gelling agent in which the value of g is 1 to 6 is most preferred. More specifically, an aqueous gelling agent in which the mass ratio of (aqueous gelling agent in which the value of g is 1 to 10) and (aqueous gelling agent in which the value of g is 0) is 95:5 to 85:15 can exhibit the advantageous effect of this invention to a remarkable extent.

Moreover, an aqueous gelling agent in which the mass ratio of (aqueous gelling agent in which the value of g is 1 to 10) and (aqueous gelling agent in which the value of g is 0) is 95:5 to 85:15 can give a soft gel which has particularly good self-leveling properties and elasticity that enables use in a spray bottle, and can be advantageously used in a hair cosmetic requiring these effects. Moreover, "self-leveling properties" means the property of naturally returning to a level surface after a physical impact is applied to a gel (for example, after a gel is scooped out or stirred). In addition, "able to be used in a spray bottle" means a soft gel state that exhibits elasticity when housed in a spray bottle and is easily sprayed like water when sprayed from the spray bottle (when a shear stress is applied to the gel).

Component (A) contained in the hair cosmetic composition of this invention is an aqueous gelling agent represented by general formula (1), for which the viscosity of a 1 mass% aqueous solution at 25°C is 1,000 to 5,000 mPa·s, the clouding point of the 1 mass% aqueous solution containing is 60°C to 80°C, and the weight average molecular weight is 10,000 to 30,000. The method for producing component (A) must be a method by which component (A) having the properties mentioned above is obtained. This component can be synthesized in the presence of a specific catalyst using compounds represented by general formulae (2) to (4) below as raw materials.

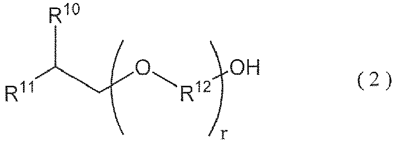

In the formula, R¹⁰ and R¹¹ each independently denote a hydrocarbon group having 4 to 20 carbon atoms, R¹² denotes a divalent hydrocarbon group having 2 to 4 carbon atoms, and r denotes a number from 10 to 100.

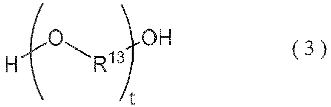

In the formula, R¹³ denotes a divalent hydrocarbon group having 2 to 4 carbon atoms, and t denotes a number from 100 to 500.

OCN-Q-NCO (4)

In the formula, Q denotes a divalent hydrocarbon group having 3 to 16 carbon atoms.

In general formula (2), R¹⁰ and R¹¹ each independently denote a hydrocarbon group having 4 to 20 carbon atoms. Examples of such a hydrocarbon group include saturated aliphatic hydrocarbon groups such as an n-butyl group, an isobutyl group, an s-butyl group, a t-butyl group, an n-pentyl group, a branched pentyl group, a secondary pentyl group, a tertiary pentyl group, an n-hexyl group, a branched hexyl group, a secondary hexyl group, a tertiary hexyl group, an n-heptyl group, a branched heptyl group, a secondary heptyl group, a tertiary heptyl group, an n-octyl group, a 2-ethylhexyl group, a branched octyl group, a secondary octyl group, a tertiary octyl group, an n-nonyl group, a branched nonyl group, a secondary nonyl group, a tertiary nonyl group, an n-decyl group, a branched decyl group, a secondary decyl group, a tertiary decyl group, an n-undecyl group, a branched undecyl group, a secondary undecyl group, a tertiary undecyl group, an n-dodecyl group, a branched dodecyl group, a secondary dodecyl group, a tertiary dodecyl group, an n-tridecyl group, a branched tridecyl group, a secondary tridecyl group, a tertiary tridecyl group, an n-tetradecyl group, a branched tetradecyl group, a secondary tetradecyl group, a tertiary tetradecyl group, an n-pentadecyl group, a branched pentadecyl group, a secondary pentadecyl group, a tertiary pentadecyl group, an n-hexadecyl group, a branched hexadecyl group, a secondary hexadecyl group, a tertiary hexadecyl group, an n-heptadecyl group, a branched heptadecyl group, a secondary heptadecyl group, a tertiary heptadecyl group, an n-octadecyl group, a branched octadecyl group, a secondary octadecyl group, a tertiary octadecyl group, an n-nonadecyl group, a branched nonadecyl group, a secondary nonadecyl group, a tertiary nonadecyl group, an n-icosyl group, a branched icosyl group, a secondary icosyl group, and a tertiary icosyl group; and unsaturated aliphatic hydrocarbon groups such as a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group, a 1-heptenyl group, a 6-heptenyl group, a 1-octenyl group, a 7-octenyl group, a 8-nonenyl group, a 1-decenyl group, a 9-decenyl group, a 10-undecenyl group, a 1-dodecenyl group, a 4-dodecenyl group, a 11-dodecenyl group, a 12-tridecenyl group, a 13-tetradecenyl group, a 14-pentadecenyl group, a 15-hexadecenyl group, a 16-heptadecenyl group, a 1-octadecenyl group, a 17-octadecenyl group, a 1-nonadecenyl group, and a 1-icosenyl group.

aromatic hydrocarbon groups such as a phenyl group, a toluyl group, a xylyl group, a cumenyl group, a mesityl group, a benzyl group, a phenethyl group, a styryl group, a cinnamyl group, a benzhydryl group, a trityl group, an ethylphenyl group, a propylphenyl group, a butylphenyl group, a pentylphenyl group, a hexylphenyl group, a heptylphenyl group, an octylphenyl groups, a nonylphenyl group, a decylphenyl group, an undecylphenyl group, a dodecylphenyl group, a styrenated phenyl group, a p-cumylphenyl group, a phenylphenyl group, a benzylphenyl group, an α-naphthyl group, and a β-naphthyl group; and alicyclic hydrocarbon groups such as a cyclopropyl group, a cyclobutyl group, a cyclopentyl group, a cyclohexyl group, a cycloheptyl group, a cyclooctyl group, a methylcyclopentyl group, a methylcyclohexyl group, a methylcycloheptyl group, a methylcyclooctyl group, a 4,4,6,6-tetramethylcyclohexyl group, a 1,3-dibutylcyclohexyl group, a norbornyl group, a bicyclo[2.2.2]octyl group, an adamantyl group, a 1-cyclobutenyl group, a 1-cyclopentenyl group, a 3-cyclopentenyl group, a 1-cyclohexenyl group, a 3-cyclohexenyl group, a 3-cycloheptenyl group, a 4-cyclooctenyl group, a 2-methyl-3-cyclohexenyl group, and a 3,4-dimethyl-3-cyclohexenyl group.

R¹⁰ and R¹¹ may be the same as, or different from, each other. Of these, saturated aliphatic hydrocarbon groups and unsaturated aliphatic hydrocarbon groups are preferred, saturated aliphatic hydrocarbon groups are more preferred, saturated aliphatic hydrocarbon groups having 5 to 18 carbon atoms are further preferred, saturated aliphatic hydrocarbon groups having 8 to 14 carbon atoms are yet further preferred, and saturated aliphatic hydrocarbon groups having 10 to 12 carbon atoms are most preferred from the perspectives of readily achieving the advantageous effect of this invention, ease of procurement of raw materials and ease of production.

In general formula (2), R¹² denotes a divalent hydrocarbon group having 2 to 4 carbon atoms. Examples of such a hydrocarbon group include an ethylene group; a propane-1,3-diyl (linear propylene) group; a branched propylene group such as a propane-1,2-diyl group and a propane-2,2-diyl group; linear butylene groups such as a butane-1,4-diyl group, a butane-1,2-diyl group, a butane-1,3-diyl group, a butane-2,3-diyl group, a butane-1,1-diyl group, and a butane-2,2-diyl group; and branched butylene groups such as a 2-methylpropane-1,3-diyl group and a 2-methylpropane-1,2-diyl group. Of these, linear divalent hydrocarbon groups having 2 to 4 carbon atoms are preferred, an ethylene group and a propane-1,3-diyl (linear propylene) group are more preferred, and an ethylene group is further preferred from the perspective of readily achieving the advantageous effect of this invention. Moreover, the R¹² groups may all be the same as, or different from, each other.

r denotes a number from 10 to 100, and within this range, the value of r is preferably a number from 12 to 50, and more preferably a number from 15 to 30, from the perspective of ease of procurement or production of a compound represented by general formula (2).

In general formula (3), R¹³ denotes a divalent hydrocarbon group having 2 to 4 carbon atoms. Examples of such hydrocarbon groups include an ethylene group; a propane-1,3-diyl (linear propylene) group; branched propylene groups such as a propane-1,2-diyl group and a propane-2,2-diyl group; linear butylene groups such as a butane-1,4-diyl group, a butane-1,2-diyl group, a butane-1,3-diyl group, a butane-2,3-diyl group, a butane-1,1-diyl group and a butane-2,2-diyl group; and branched butylene groups such as 2-methylpropane-1,3-diyl group and a 2-methylpropane-1,2-diyl group. Of these, linear divalent hydrocarbon groups having 2 to 4 carbon atoms are preferred, an ethylene group and a propane-1,3-diyl (linear propylene) group are more preferred, and an ethylene group is further preferred from the perspective of readily achieving the advantageous effect of this invention. Moreover, the R¹³ groups may all be the same as, or different from, each other.

t denotes a number from 100 to 500, and within this range, the value of t is preferably 120 to 450, more preferably 150 to 400, further preferably 180 to 350, and most preferably 200 to 300 from the perspective of readily achieving the advantageous effect of this invention.

Examples of diisocyanate compounds represented by general formula (4) include aliphatic diisocyanates such as trimethylene diisocyanate, tetramethylene diisocyanate, pentamethylene diisocyanate, hexamethylene diisocyanate (HDI), 2,2-dimethylpentane diisocyanate, octamethylene diisocyanate, 2,2,4-trimethylpentane diisocyanate, nonamethylene diisocyanate, decamethylene diisocyanate, dodecamethylene diisocyanate, isophorone diisocyanate (IPDI), dicyclohexylmethane diisocyanate (hydrogenated MDI), hydrogenated xylylene diisocyanate (hydrogenated XDI) and 2,4,4 (or 2,2,4)-trimethylhexamethylene diisocyanate (TMDI); and aromatic diisocyanates such as tolylene diisocyanate (TDI), diphenylmethane diisocyanate (MDI), toluidene diisocyanate (TODI), xylylene diisocyanate (XDI) and naphthalene diisocyanate (NDI).

In general formula (4), Q may be any divalent hydrocarbon group having 3 to 16 carbon atoms, but a group obtained by removing two isocyanate groups from the diisocyanate compounds listed above is preferred. Among the diisocyanate compounds, aliphatic diisocyanates are preferred, trimethylene diisocyanate, tetramethylene diisocyanate, hexamethylene diisocyanate (HDI), octamethylene diisocyanate, isophorone diisocyanate (IPDI), dicyclohexylmethane diisocyanate (hydrogenated MDI), hydrogenated xylylene diisocyanate (hydrogenated XDI) and 2,4,4 (or 2,2,4)-trimethylhexamethylene diisocyanate (TMDI) are more preferred, tetramethylene diisocyanate, hexamethylene diisocyanate (HDI), isophorone diisocyanate (IPDI) and dicyclohexylmethane diisocyanate (hydrogenated MDI) are further preferred, and hexamethylene diisocyanate (HDI) is most preferred.

Moreover, in general formula (1) above, R⁴ and R⁶ each independently denote a divalent hydrocarbon group having 3 to 16 carbon atoms. More specifically, groups obtained by removing two isocyanate groups from the diisocyanate compounds listed above are preferred, groups obtained by removing two isocyanate groups from aliphatic diisocyanates are more preferred, groups obtained by removing two isocyanate groups from any of trimethylene diisocyanate, tetramethylene diisocyanate, hexamethylene diisocyanate (HDI), octamethylene diisocyanate, isophorone diisocyanate (IPDI), dicyclohexylmethane diisocyanate (hydrogenated MDI), hydrogenated xylylene diisocyanate (hydrogenated XDI) and 2,4,4 (or 2,2,4)-trimethylhexamethylene diisocyanate (TMDI) are further preferred, groups obtained by removing two isocyanate groups from any of tetramethylene diisocyanate, hexamethylene diisocyanate (HDI), isophorone diisocyanate (IPDI) and dicyclohexylmethane diisocyanate (hydrogenated MDI) are yet further preferred, and a group obtained by removing two isocyanate groups from hexamethylene diisocyanate (HDI) is most preferred.

Examples of specific catalysts used when producing component (A) include higher fatty acid metal salts selected from among lauric acid metal salts, myristic acid metal salts, palmitic acid metal salts, stearic acid metal salts and oleic acid metal salts, and it is possible to use one or more types of these. Moreover, metal salts means any of calcium salts, potassium salts, sodium salts and magnesium salts. Of these, lauric acid metal salts are preferred, and potassium laurate and sodium laurate are more preferred, from the perspective of being able to produce an aqueous gelling agent that exhibits the advantageous effect of this invention to a remarkable extent.

In general, an aqueous gelling agent represented by general formula (1) can be produced with or without using a catalyst. In cases where a catalyst is used, it is possible to use a metal halide such as titanium tetrachloride, hafnium chloride, zirconium chloride, aluminum chloride, gallium chloride, indium chloride, iron chloride, tin chloride or boron fluoride; an alkali metal or alkaline metal hydroxide, alcoholate or carbonate, such as sodium hydroxide, potassium hydroxide, sodium methylate, or sodium carbonate; a metal oxide such as aluminum oxide, calcium oxide, barium oxide or sodium oxide; an organometallic compound such as tetraisopropyl titanate, dibutyltin dichloride, dibutyltin oxide, dibutyltin dilaurate or dibutyltin bis(2-ethylhexylthioglycolate); or a soap such as sodium octylate or potassium octylate. However, an aqueous gelling agent represented by general formula (1), for which the viscosity of an aqueous solution containing 1 mass% of component (A) at 25°C is 1,000 to 5,000 mPa·s, the clouding point of the aqueous solution containing 1 mass% of component (A) is 60°C to 80°C, and the weight average molecular weight of component (A) is 10,000 to 30,000, can be obtained using the higher fatty acid metal salts mentioned above. In cases where a catalyst is not used or in cases where a catalyst other than a higher fatty acid metal salt is used, the viscosity of a 1 mass% aqueous solution, the clouding point of a 1 mass% aqueous solution and the weight average molecular weight might, in some cases, deviate from the specifications of component (A) of this invention.

A higher fatty acid metal salt can be used as a catalyst at a quantity of 0.01 to 5 mass% relative to the overall quantity of the reaction system for the production of component (A). Within this range, the higher fatty acid metal salt is preferably used at a quantity of 0.1 to 2 mass%, and more preferably 0.5 to 1 mass%, relative to the overall reaction system from the perspective of satisfactorily achieving a significant advantageous effect. If this quantity is less than 0.01 mass%, the function of the catalyst is not sufficiently exhibited and component (A) used in this invention might not be obtained, and if this quantity exceeds 5 mass%, an advantageous effect commensurate with the added quantity might not be achieved. Moreover, a removal step is not carried out following production of component (A) in this invention, and the catalyst remains in the hair cosmetic containing components (A) and (B) of this invention, and the remaining quantity of catalyst depends on the usage quantity of the catalyst in the production of component (A) and the blending quantity of component (A) in the hair cosmetic composition described below.

A method for synthesizing component (A) in the presence of a specific catalyst using compounds represented by general formulae (2) to (4) listed above as raw materials can be given as an example of a method for producing component (A). For example, a reaction should be carried out after adding 1.5 to 2.4 moles, preferably 1.8 to 2.2 moles, and more preferably 1.9 to 2.1 moles, of an alcohol compound represented by general formula (2), 0.5 to 1.4 moles, preferably 0.8 to 1.2 moles, and more preferably 0.9 to 1.1 moles, of a polyalkylene glycol represented by general formula (3) and a catalyst to 2 moles of a diisocyanate compound represented by general formula (4). An example of a method includes one in which specific reaction conditions are such that the diisocyanate compound represented by general formula (4), the alcohol compound represented by general formula (2) and the polyalkylene glycol represented by general formula (3) are added to the system together with the catalyst and allowed to react for 1 to 10 hours at 60°C to 100°C. An example of a method includes one in which more specific reaction conditions are such that a system containing the alcohol compound represented by general formula (2) and the polyalkylene glycol represented by general formula (3) is homogeneously mixed, after which the diisocyanate compound represented by general formula (4) and the catalyst are added and allowed to react for 1 to 10 hours at 60°C to 100°C.

The viscosity of an aqueous solution containing 1 mass% of component (A), which constitutes the hair cosmetic composition of this invention, at 25°C is 1,000 to 5,000 mPa·s. Within this range, the viscosity is preferably 1,200 to 4,000 mPa·s, and more preferably 1,500 to 3,500 mPa·s, from the perspective of achieving the advantageous effect of this invention to a more remarkable extent. A viscosity measurement method using a B type viscometer at 25°C, as described in JIS Z 8803: 2011, can be used as a method for measuring the viscosity of the 1 mass% aqueous solution at 25°C.

The clouding point of the aqueous solution containing 1 mass% of component (A), which is contained in the hair cosmetic composition of this invention, is 60°C to 80°C. Within this range, the clouding point is preferably 60°C to 70°C from the perspective of achieving the advantageous effect of this invention to a more remarkable extent. A method comprising preparing a 1 mass% aqueous solution of component (A), gradually increasing the temperature of the aqueous solution, and taking the clouding point to be the temperature at which turbidity occurs can be used as a method for measuring the clouding point.

The weight average molecular weight of component (A), which is contained in the hair cosmetic composition of this invention, is 10,000 to 30,000. Within this range, the weight average molecular weight is preferably 12,000 to 28,000, and more preferably 18,000 to 25,000, from the perspective of achieving the advantageous effect of this invention to a more remarkable extent. A method comprising measuring the weight average molecular weight in terms of standard polystyrene using gel permeation chromatography (GPC) can be used as a method for measuring the weight average molecular weight. In this invention, by incorporating component (A) whose weight average molecular weight falls within this range, it is possible to obtain a hair cosmetic composition that satisfies all of the advantageous effects of this invention, and possible to obtain, in particular, a hair cosmetic composition that imparts a favorable texture to hair when applied thereto.

Component (A) contained in the hair cosmetic composition of this invention is a solid or viscous substance at room temperature. From the perspective of ease of handling when blended in a hair cosmetic, it is preferable to first dissolve component (A) in a solvent such as water so as to obtain a liquid. The amount of solvent is not particularly limited, but from the perspective of ease of handling, this quantity is preferably such that the content of component (A) is 10 to 50 mass%, and more preferably 15 to 40 mass%.

Examples of solvents able to be used include water and volatile primary alcohol compounds such as methanol, ethanol and propanol. Meanwhile, because use of volatile solvents may be restricted according to the site of use, water is most preferred among these solvents.

The blending quantity of component (A) that constitutes the hair cosmetic composition of this invention is not particularly limited, but is preferably 0.05 to 30 mass%, more preferably 0.1 to 20 mass%, further preferably 0.3 to 10 mass%, and most preferably 0.5 to 5 mass%, relative to the overall quantity of the hair cosmetic composition in order to form an aqueous gel that can readily achieve the advantageous effect of this invention.

Component (B) contained in the hair cosmetic composition of this invention is either one of or both of a cationic surfactant and a cationic polymer. The cationic surfactant is not particularly limited as long as this component is a cationic surfactant well-known in the cosmetic industry, but examples of cationic surfactants suitable for the hair cosmetic composition of this invention include alkyltrimethyl ammonium salts represented by general formula (5), alkyltriethyl ammonium salts represented by general formula (6), dialkyldimethyl ammonium salts represented by general formula (7), alkoxyalkyltrimethyl ammonium salts represented by general formula (8), salts produced by reacting an alkyldimethylamine with an acid, salts produced by reacting an alkoxydimethylamine with an acid, and amide compounds represented by general formula (11).

The above-mentioned alkyltrimethyl ammonium salts represented by general formula (5) are as follows.

R¹⁴-N⁺(CH₃)₃X⁻ (5)

In the formula, R¹⁴ denotes an alkyl group having 10 to 24 carbon atoms, and X- denotes a halide ion, such as a chloride ion or bromide ion, a methosulfate ion, an ethosulfate ion, a methophosphate ion, an ethophosphate ion, a methocarbonate ion, or the like.

Specific examples thereof include cetyltrimethyl ammonium chloride, stearyltrimethyl ammonium chloride, lauryltrimethyl ammonium chloride, behenyltrimethyl ammonium chloride and behenyltrimethyl ammonium methosulfate.

The above-mentioned alkyltriethyl ammonium salts represented by general formula (6) are as follows.

R¹⁵-N⁺(C₂H₅)₃X⁻ (6)

In the formula, R¹⁵ denotes an alkyl group having 10 to 24 carbon atoms, and X⁻ denotes a halide ion, such as a chloride ion or bromide ion, a methosulfate ion, an ethosulfate ion, a methophosphate ion, an ethophosphate ion, a methocarbonate ion, or the like.

Specific examples thereof include cetyltriethyl ammonium chloride, stearyltriethyl ammonium chloride, lauryltriethyl ammonium chloride, behenyltriethyl ammonium chloride, cetyltriethyl ammonium methosulfate and behenyltriethyl ammonium methosulfate.

The above-mentioned dialkyldimethyl ammonium salts represented by general formula (7) are as follows.

(R¹⁶)₂N⁺(CH₃)₂X⁻ (7)

In the formula, the R¹⁶ groups each independently denote an alkyl group having 10 to 24 carbon atoms or a benzyl group, and X- denotes a halide ion such as a chloride ion or bromide ion, a methosulfate ion, an ethosulfate ion, a methophosphate ion, an ethophosphate ion, a methocarbonate ion, or the like.

Specific examples thereof include distearyldimethyl ammonium chloride, dicetyldimethyl ammonium chloride, dilauryldimethyl ammonium chloride and stearyldimethylbenzyl ammonium chloride.

The above-mentioned alkoxyalkyltrimethyl ammonium salts represented by general formula (8) are as follows.

R¹⁷-O-R¹⁸-N⁺(CH₃)₃X⁻ (8)

In the formula, R¹⁷ denotes an alkyl group having 10 to 24 carbon atoms, R¹⁸ denotes an ethylene group or propylene group that may be substituted with a hydroxyl group, and X⁻ denotes a halide ion, such as a chloride ion or bromide ion, a methosulfate ion, an ethosulfate ion, a methophosphate ion, an ethophosphate ion, a methocarbonate ion, or the like.

Specific examples thereof include stearoxypropyltrimethyl ammonium chloride, stearoxyethyltrimethyl ammonium chloride and stearoxyhydroxypropyltrimethyl ammonium chloride.

Examples of salts produced by reacting an alkyldimethylamine with an acid include salts obtained from an alkyldimethylamine represented by general formula (9) and an organic acid or inorganic acid. The alkyldimethylamine represented by general formula (9) is as follows.

R¹⁹-N(CH₃)₂ (9)

In the formula, R¹⁹ denotes an alkyl group having 10 to 24 carbon atoms.

Specific examples thereof include N,N-dimethylbehenylamine and N,N-dimethylstearylamine.

Examples of organic acids include monocarboxylic acids such as acetic acid and propionic acid; dicarboxylic acids such as malonic acid, succinic acid, glutaric acid, adipic acid, maleic acid, fumaric acid and phthalic acid; polycarboxylic acids such as poly(glutamic acid); hydroxycarboxylic acids such as glycolic acid, lactic acid, hydroxyacrylic acid, glyceric acid, malic acid, tartaric acid and citric acid; and acidic amino acids such as glutamic acid and aspartic acid.

Examples of inorganic acids include hydrochloric acid, sulfuric acid and phosphoric acid.

Examples of salts produced by reacting an alkoxydimethylamine with an acid include salts obtained from an alkoxydimethylamine represented by general formula (10) and an organic acid or inorganic acid. The alkoxydimethylamine represented by general formula (10) is as follows.

R²⁰-O-R²¹-N(CH₃)₂ (10)

In the formula, R²⁰ denotes an alkyl group having 10 to 24 carbon atoms, and R²¹ denotes an ethylene group or propylene group that may be substituted with a hydroxyl group.

Specific examples thereof include N,N-dimethyl-3-hexadecyloxypropylamine and N,N-dimethyl-3-octadecyloxypropylamine.

Examples of organic acids include monocarboxylic acids such as acetic acid and propionic acid; dicarboxylic acids such as malonic acid, succinic acid, glutaric acid, adipic acid, maleic acid, fumaric acid and phthalic acid; polycarboxylic acids such as poly(glutamic acid); hydroxycarboxylic acids such as glycolic acid, lactic acid, hydroxyacrylic acid, glyceric acid, malic acid, tartaric acid and citric acid; and acidic amino acids such as glutamic acid and aspartic acid.

Examples of inorganic acids include hydrochloric acid, sulfuric acid and phosphoric acid.

The above-mentioned amide compound represented by general formula (11) is as follows.

R²²-CONH-(R²³)-N-(R²⁴)₂ (11)

In the formula, R²² denotes an alkyl group having 10 to 24 carbon atoms, R²³ denotes an alkylene group having 2 to 4 carbon atoms, and the R²⁴ moieties each independently denote an alkyl group having 1 to 4 carbon atoms.

Specific examples thereof include diethylaminoethylamide stearate, dimethylaminoethylamide stearate, diethylaminoethylamide palmitate, dimethylaminoethylamide palmitate, diethylaminoethylamide myristate, dimethylaminoethylamide myristate, diethylaminoethylamide behenate, dimethylaminoethylamide behenate, diethylaminopropylamide stearate, dimethylaminopropylamide stearate, diethylaminopropylamide palmitate, dimethylaminopropylamide palmitate, diethylaminopropylamide myristate, dimethylaminopropylamide myristate, diethylaminopropylamide behenate and dimethylaminopropylamide behenate.

Of these, it is preferable to select either one of or both of an alkyltrimethyl ammonium salt represented by general formula (5) and an amide compound represented by general formula (11) from the perspective of readily achieving the advantageous effect of this invention, and especially obtaining a hair cosmetic composition that imparts a favorable texture to hair when applied thereto when component (B) is combined with component (A).

The cationic polymer is not particularly limited as long as this is a polymer compound which is well known in the cosmetic industry, has a quaternary nitrogen-containing group and exhibits cationic properties. However, examples of cationic polymers suitable for the hair cosmetic composition of this invention include cationized cellulose and derivatives thereof, cationized guar gum and derivatives thereof, cationized silicones and derivatives thereof, cationic starch, diallyl quaternary ammonium salt/acrylamide copolymers, and quaternized polyvinylpyrrolidone and derivatives thereof.

Specific examples thereof include Silicone Quaternium-13, Silicone Quaternium-15, Silicone Quaternium-16, Silicone Quaternium-17, Silicone Quaternium-18, Silicone Quaternium-22, Silicone Quaternium-25, Silicone Quaternium-26, Silicone Quaternium-3, Silicone Quaternium-5, Silicone Quaternium-8, Polyquaternium-1, Polyquaternium-10, Polyquaternium-102, Polyquaternium-103, Polyquaternium-104, Polyquaternium-105, Polyquaternium-106, Polyquaternium-107, Polyquaternium-11, Polyquaternium-111, Polyquaternium-112, Polyquaternium-113, Polyquaternium-16, Polyquaternium-2, Polyquaternium-22, Polyquaternium-24, Polyquaternium-28, Polyquaternium-30, Polyquaternium-32, Polyquaternium-33, Polyquaternium-37, Polyquaternium-39, Polyquaternium-4, Polyquaternium-43, Polyquaternium-44, Polyquaternium-46, Polyquaternium-47, Polyquaternium-48, Polyquaternium-49, Polyquaternium-5, Polyquaternium-50, Polyquaternium-51, Polyquaternium-52, Polyquaternium-53, Polyquaternium-54, Polyquaternium-55, Polyquaternium-56, Polyquaternium-57, Polyquaternium-59, Polyquaternium-6, Polyquaternium-61, Polyquaternium-62, Polyquaternium-63, Polyquaternium-64, Polyquaternium-65, Polyquaternium-66, Polyquaternium-67, Polyquaternium-68, Polyquaternium-69, Polyquaternium-7, Polyquaternium-70, Polyquaternium-72, Polyquaternium-73, Polyquaternium-74, Polyquaternium-75, Polyquaternium-76, Polyquaternium-77, Polyquaternium-84, Polyquaternium-85, Polyquaternium-86, Polyquaternium-87, Polyquaternium-90, Polyquaternium-91, Polyquaternium-92, Polyquaternium-94 and Polyquaternium-99. Of these, Polyquaternium-6 is preferred from the perspective of achieving the advantageous effect of this invention when component (B) is combined with component (A) .

Moreover, the cationic surfactant or cationic polymer of component (B) can achieve the advantageous effect of this invention when either one of the two is used in combination with component (A). However, by using both the cationic surfactant and cationic polymer of component (B) in combination with component (A), flow properties and texture of hair when applied thereto, which are advantageous effects of this invention, are synergistically improved. Therefore, it is preferable to use both the cationic surfactant and cationic polymer of component (B).

It is possible to blend one or more cationic surfactants and cationic polymers of component (B), which constitutes the hair cosmetic composition of this invention. The blending quantity thereof is not particularly limited, but is preferably 0.01 to 20 mass%, more preferably 0.05 to 10 mass%, further preferably 0.1 to 5 mass%, and most preferably 0.5 to 1 mass%, relative to the overall quantity of the hair cosmetic composition from the perspective of readily achieving the advantageous effect of this invention.

The blending ratio (mass ratio) of component (A) and component (B) is not particularly limited, but it is preferable for the mass ratio of component (A) to component (B) to be 1:0.02 to 1:50 from the perspectives of better achieving the gelling effect of component (A) and achieving flow properties suitable for a hair cosmetic. Here, in the case of an aqueous cosmetic in which component (B) is a cationic surfactant and the content of oily components is 5 mass% or less, the component (A) : component (B) blending ratio is more preferably 1:0.02 to 1:1, further preferably 1:0.05 to 1:1, and yet more preferably 1:0.1 to 1:1. In this invention, oily component means the liquid oils/fats, ester oils, silicone oils, solid oils/fats, waxes, hydrocarbon oils and higher fatty acids mentioned below, which are commonly added to cosmetic compositions.

In the case of an aqueous cosmetic in which component (B) is a cationic polymer or a mixture of a cationic surfactant and a cationic polymer and the content of oily components is 5 mass% or less and the case of an oil-based cosmetic in which the content of oily components exceeds 5 mass % regardless of the type of component (B), the component (A) : component (B) blending ratio is more preferably 1:1 to 1:50, further preferably 1:1 to 1:30, and yet more preferably 1:1 to 1:10.

The hair cosmetic composition of this invention may contain other additives that are commonly used in cosmetic compositions in order to impart a variety of characteristics as appropriate within qualitative and quantitative ranges that do not impair the advantageous effect of this invention. Examples thereof include powder components, liquid oils/fats, ester oils, silicone oils, solid oils/fats, waxes, hydrocarbon oils, higher fatty acids, higher alcohols, polyol compounds, non-ionic surfactants, anionic surfactants, amphoteric surfactants, humectants, water-soluble polymer compounds, metal ion sequestering agents, sugars, amino acids and derivatives thereof, organic amines, pH adjusting agents, antioxidants, preservatives, blood circulation promoters, antiphlogistic agents, activators, whitening agents, antiseborrheic agents, anti-inflammatory agents, and a variety of extracts, and one or more types of these can be blended according to need.

Examples of powder components include inorganic powders (for example, talc, kaolin, mica, sericite, muscovite, phlogopite, synthetic mica, red mica, biotite, vermiculite, magnesium carbonate, calcium carbonate, aluminum silicate, barium silicate, calcium silicate, magnesium silicate, strontium silicate, metal tungstates, magnesium, silica, zeolites, barium sulfate, calcined calcium sulfate (calcined gypsum), calcium phosphate, fluorapatite, hydroxyapatite, ceramic powders, metal soaps (for example, zinc myristate, calcium palmitate and aluminum stearate), boron nitride, and the like); organic powders (for example, polyamide resin powders (nylon powders), polyethylene powders, poly(methyl methacrylate) powders, polystyrene powders, styrene-acrylic acid copolymer resin powders, benzoguanamine resin powders, polytetrafluoroethylene powders, cellulose powders, and the like); inorganic white pigments (for example, titanium dioxide, zinc oxide, and the like); inorganic red pigments (for example, iron oxide (red iron oxide), iron titanate, and the like), inorganic brown pigments (for example, γ-iron oxide and the like); inorganic yellow pigments (for example, yellow iron oxide, loess, and the like); inorganic black pigments (for example, black iron oxide, lower-order titanium oxides, and the like); inorganic violet pigments (for example, manganese violet, cobalt violet, and the like), inorganic green pigments (for example, chromium oxide, chromium hydroxide, cobalt titanate, and the like); inorganic blue pigments (for example, ultramarine blue, Prussian blue, and the like); pearlescent pigments (for example, titanium oxide-coated mica, titanium oxide-coated bismuth oxychloride, titanium oxide-coated talc, colored titanium oxide-coated mica, bismuth oxychloride, fish scales, and the like), metal powder pigments (for example, aluminum powders, copper powders, and the like); organic pigments such as zirconium, barium and aluminum lakes (for example, organic pigments such as Red 201, Red 202, Red 204, Red 205, Red 220, Red 226, Red 228, Red 405, Orange 203, Orange 204, Yellow 205, Yellow 401 and Blue 404; Red 3, Red 104, Red 106, Red 227, Red 230, Red 401, Red 505, Orange 205, Yellow 4, Yellow 5, Yellow 202, Yellow 203, Green 3, Blue 1, and the like); and natural dyes (for example, chlorophyll, β-carotene, and the like).

Examples of liquid oils/fats include avocado oil, camellia oil, turtle oil, macadamia nut oil, corn oil, mink oil, olive oil, rape seed oil, egg yolk oil, sesame oil, persic oil, wheat germ oil, sasanqua oil, castor oil, linseed oil, safflower oil, cottonseed oil, perilla oil, soy bean oil, peanut oil, tea seed oil, kaya oil, rice bran oil, Chinese tung oil, Japanese tung oil, jojoba oil, germ oil, and triglycerides.

Examples of ester oils include isopropyl myristate, octyldodecyl myristate, isopropyl isostearate, isononyl isononanoate, isotridecyl isononanoate, butyl stearate, oleyl oleate, octyldodecyl ricinoleate, octyl hydroxystearate, ethylhexyl para-methoxycinnamate, neopentyl glycol dicaprate, propylene glycol dicaprate, diisostearyl malate, polyglyceryl diisostearate, polyglyceryl triisostearate, glyceryl diisostearate, glyceryl triisostearate, glyceryl tri(caprylate/caprate), glyceryl trihexanoate, glyceryl tri-2-ethylhexanoate, trimethylolpropane tri-2-ethylhexanoate, trimethylolpropane triisostearate, dl-α-tocopherol, dl-α-tocopherol nicotinate, pentaerythrityl tetraoctanoate, and dipentaerythrityl tripolyhydroxystearate.

Examples of silicone oils include polysiloxanes, dimethylpolysiloxanes, dimethicone, methylphenylpolysiloxanes, cyclic dimethicone, amino-modified silicones, carbinol-modified silicones, methacrylic-modified silicones, mercaptomodified silicones, phenol-modified silicones, polyethermodified silicones, methylstyryl-modified silicones, alkylmodified silicones, and higher fatty acid ester-modified silicones.

Examples of solid oils/fats include cocoa butter, coconut oil, hydrogenated coconut oil, palm oil, palm kernel oil, Japan wax kernel oil, hydrogenated oils, Japan wax, and hydrogenated castor oil.

Examples of waxes include beeswax, candelilla wax, cotton wax, carnauba wax, bayberry wax, insect wax, spermaceti, montan wax, bran wax, lanolin, kapok wax, lanolin acetate, liquid lanolin, sugar cane wax, isopropyl lanolin fatty acids, hexyl laurate, reduced lanolin, jojoba wax, hard lanolin, shellac wax, POE lanolin alcohol ethers, POE lanolin alcohol acetates, POE cholesterol ethers, polyethylene glycol lanolate, and POE hydrogenated lanolin alcohol ethers.

Examples of hydrocarbon oils include liquid paraffin, ozokerite, squalane, pristane, paraffin, ceresin, squalene, Vaseline, and microcrystalline waxes.

Examples of higher fatty acids include decanoic acid, lauric acid, myristic acid, palmitic acid, stearic acid, behenic acid, oleic acid, undecylenic acid, tall oil fatty acids, 12-hydroxystearic acid, isostearic acid, linolic acid, linolenic acid, eicosapentaenoic acid (EPA), and docosahexaenoic acid (DHA).

Examples of higher alcohols include linear alcohols such as decyl alcohol, lauryl alcohol, cetyl alcohol, stearyl alcohol, behenyl alcohol, myristyl alcohol, oleyl alcohol, and cetostearyl alcohol; and branched alcohols such as monostearyl glycerin ether (batyl alcohol), 2-decyltetradecyl alcohol, lanolin alcohol, cholesterol, phytosterols, hexyldodecanol, isostearyl alcohol, and octyldodecanol.

Examples of polyol compounds include ethylene glycol, propylene glycol, butylene glycol, glycerin, diethylene glycol, dipropylene glycol, and sugar alcohols.

Examples of non-ionic surfactants include sorbitan fatty acid esters (for example, sorbitan monooleate, sorbitan monoisostearate, sorbitan monolaurate, sorbitan monopalmitate, sorbitan monostearate, sorbitan sesquiolate, sorbitan trioleate, diglycerol sorbitan penta-2-ethylhexanoate, diglycerol sorbitan tetra-2-ethylhexanoate, and the like); glycerol/polyglycerol fatty acids (for example, glycerol mono-cottonseed oil fatty acids, glycerol monoerucate, glycerol sesquioleate, glycerol monostearate, glycerol POE-monostearate, polyglycerol monoisostearate, glycerol α,α'-oleate pyroglutamate, glycerol monostearate malate, and the like); propylene glycol fatty acid esters (for example, propylene glycol monostearate and the like); hydrogenated castor oil derivatives; glycerol/polyglycerol alkyl ethers (for example, polyglyceryl/polyoxybutylene stearyl ether and the like); POE-sorbitan fatty acid esters (for example, POE-sorbitan monooleate, POE-sorbitan monostearate, POE-sorbitan tetraoleate, and the like); POE sorbitol fatty acid esters (for example, POE-sorbitol monolaurate, POE-sorbitol monooleate, POE-sorbitol pentaoleate, POE-sorbitol monostearate, and the like); POE-glycerin fatty acid esters (for example, POE-glycerin monostearate, POE-glycerin monoisostearate, POE-glycerin triisostearate); and POE-difatty acid esters (for example, POE-distearate, POE-dioleate, and the like);

POE-alkyl ethers (for example, POE-lauryl ether, POE-oleyl ether, POE-stearyl ether, POE-behenyl ether, POE-2-octyldodecyl ether, POE-cholestanol ether, and the like); pluronic surfactants (for example, Pluronic and the like); POE/POP-alkyl ethers (for example, POE/POP-lauryl ether, POE/POP-cetyl ether, POE/POP-2-decyltetradecyl ether, POE/POP-monobutyl ether, POE/POP-hydrogenated lanolin, POE/POP-glycerin ether, and the like); tetra POE/tetra POP-ethylenediamine condensates (for example, Tetronic surfactants and the like); POE-castor oil/hydrogenated castor oil derivatives (for example, POE-castor oil, POE-hydrogenated castor oil, POE-hydrogenated castor oil monoisostearate, POE-hydrogenated castor oil triisostearate, POE-hydrogenated castor oil monopyroglutamate monoisostearate diester, POE-hydrogenated castor oil maleate, and the like); POE beeswax/lanolin derivatives (for example, POE sorbitol beeswax and the like); alkanolamides (for example, coconut oil fatty acid diethanolamide, coconut oil fatty acid monoethanolamide, lauric acid monoethanolamide, fatty acid isopropanolamides, and the like); POE-propylene glycol fatty acid esters; POE alkylamines; N-methylalkylglucamides (for example, N-methyllaurylglucamide and the like); N-polyhydroxyalkyl fatty acid amides; POE-fatty acid amides; sucrose fatty acid esters (for example, sucrose monostearate, sucrose monolaurate, POE-sucrose monolaurate, and the like); alkylethoxydimethylamine oxides; and trioleylphosphonic acid. Moreover, POE is an abbreviation of polyoxyethylene, and POP is an abbreviation of polyoxypropylene.

Examples of anionic surfactants include higher fatty acid salt based surfactants, sulfonic acid salt based surfactants, sulfate ester salt based surfactants, phosphate ester salt based surfactants, and sulfosuccinic acid salt based surfactants.

Examples of higher fatty acid salt based surfactants include fatty acid soaps such as salts (potassium salts, sodium salts, triethanolamine salts, ammonium salts, and the like) of saturated or unsaturated fatty acids having 12 to 18 carbon atoms, coconut oil fatty acid, hydrogenated coconut oil fatty acid, palm oil fatty acid, hydrogenated palm oil fatty acid, beef tallow fatty acid, hydrogenated beef tallow fatty acid, and the like; alkyl ether carboxylic acid salts, alkyl allyl ether carboxylic acid salts, N-acylsarcosinic acid salts, and N-acylglutamic acid salts. Specific examples thereof include potassium laurate, sodium laurate, sodium palmitate, potassium myristate, sodium lauryl ether carboxylate, sodium N-lauroylsarcosinate, sodium N-lauroylglutamate, sodium coconut oil fatty acid glutamate, disodium N-stearoylglutamate, monosodium N-myristoyl-L-glutamate, sodium coconut oil fatty acid isethionate, and triethanolamine coconut oil fatty acid.

Examples of sulfonic acid salt based surfactants include higher fatty acid amide sulfonic acid salts, alkylbenzene sulfonic acid salts, N-acylamino sulfonic acid salts, α-olefin sulfonic acid salts, and higher fatty acid ester sulfonic acid salts. Specific examples thereof include sodium N-myristoyl-N-methyltaurine, sodium N-stearoyl-N-methyltaurine, sodium coconut oil fatty acid methyltaurine, sodium coconut oil fatty acid acylmethyltaurine, sodium laurylmethyltaurine, sodium dodecylbenzene sulfonate, triethanolamine dodecylbenzene sulfonate, and sodium N-cocoyl-N-methyltaurine.

Examples of sulfate ester type surfactants include higher alkyl sulfates, polyoxyethylene alkyl ether sulfates, higher fatty acid ester sulfates, secondary alcohol sulfates, and higher fatty acid alkylolamide sulfates. Specific examples thereof include sodium lauryl sulfate, potassium lauryl sulfate, triethanolamine polyoxyethylene lauryl sulfate, sodium polyoxyethylene lauryl sulfate, and sodium hydrogenated coconut oil fatty acid glycerin sulfate.

Examples of phosphate ester type surfactants include triethanolamine monolauryl phosphate, dipotassium monolauryl phosphate, sodium polyoxyethylene oleyl ether phosphate, and sodium polyoxyethylene stearyl ether phosphate. Examples of sulfosuccinic acid salt based surfactants include sodium polyoxyethylene alkyl sulfosuccinates, sodium di-2-ethylhexyl sulfosuccinate, sodium monolauroyl monoethanolamide polyoxyethylene sulfosuccinate, and sodium lauryl polypropylene glycol sulfosuccinate.

Examples of amphoteric surfactants include imidazoline type amphoteric surfactants, betaine type amphoteric surfactants, acyl tertiary amine oxides, and acyl tertiary phosphine oxides.

Examples of imidazoline type amphoteric surfactants include sodium 2-undecyl-N,N,N-(hydroxyethylcarboxymethyl)-2-imidazoline, and 2-cocoyl-2-imidazolinium hydroxide-1-carboxyethyloxy disodium salt.

Examples of betaine type amphoteric surfactants include alkylbetaines, alkylamidobetaines, alkylsulfobetaines, alkylhydroxysulfobetaines, and phosphobetaines. Specific examples thereof include lauryl dimethylamino acetic acid betaine, myristyl dimethylamino acetic acid betaine, coconut oil fatty acid amidopropyl betaine, coconut oil fatty acid dimethylsulfopropyl betaine, lauryldimethylaminohydroxysulfo betaine, laurylhydroxyphosphobetaine, 2-heptadecyl-N-carboxymethyl-N-hydroxyethyl imidazolinium betaine, and coconut oil alkyl-N-hydroxyethyl imidazolinium betaine.

Examples of acyl tertiary amine oxides include lauryldimethylamine oxide. Examples of acyl tertiary phosphine oxides include lauryldimethylphosphine oxide.

Examples of humectants include polyethylene glycol, xylitol, sorbitol, maltitol, L-glutamic acid, chondroitin sulfate, hyaluronic acid, mucoitin sulfate, calonic acid, atelocollagen, cholesteryl-12-hydroxystearate, sodium lactate, bile salts, dl-pyrrolidone carboxylic acid salts, short chain soluble collagen, diglycerol (EO)PO adducts, Rosa roxburghii extract, Achillea millefolium extract, and melilot extract.

Examples of water-soluble polymer compounds (excluding cationic polymers) include starch-based polymers (for example, carboxymethyl starch, methylhydroxypropyl starch, and the like); cellulose-based polymers (methyl cellulose, ethyl cellulose, methylhydroxypropyl cellulose, hydroxyethyl cellulose, sodium cellulose sulfate, hydroxypropyl cellulose, carboxymethyl cellulose, sodium carboxymethyl cellulose, crystalline cellulose, cellulose powders, and the like); alginic acid-based polymers (for example, sodium alginate, propylene glycol alginate, and the like), vinyl-based polymers (for example, poly(vinyl alcohol), poly(vinyl methyl ether), polyvinylpyrrolidone, carboxyvinyl polymers, and the like); polyoxyethylene-based polymers (for example, polyoxyethylene-polyoxypropylene copolymers prepared from polyethylene glycol 20,000, 40,000 or 60,000 and the like); and acrylic-based polymers (for example, sodium polyacrylate, poly(ethyl acrylate), polyacrylamide, and the like).

Examples of metal ion-sequestering agents include 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid and trisodium ethylenediamine hydroxyethyl triacetate.

Examples of monosaccharides include trioses (for example, D-glyceryl aldehyde, dihydroxyacetone, and the like); tetroses (for example, D-erythrose, D-erythrulose, D-threose, erythritol, and the like); pentoses (for example, L-arabinose, D-xylose, L-lyxose, D-arabinose, D-ribose, D-ribulose, D-xylulose, L-xylulose, and the like); hexoses (for example, D-glucose, D-talose, D-psicose, D-galactose, D-fructose, L-galactose, L-mannose, D-tagatose, and the like); heptoses (for example, aldoheptose, hepulose, and the like); octoses (for example, octulose and the like); deoxy sugars (for example, 2-deoxy-D-ribose, 6-deoxy-L-galactose, 6-deoxy-L-mannose, and the like); amino sugars (for example, D-glucosamine, D-galactosamine, sialic acid, aminouronic acid, muramic acid, and the like); uronic acids (for example, D-glucuronic acid, D-mannuronic acid, L-guluronic acid, D-galacturonic acid, L-iduronic acid, and the like).

Examples of oligosaccharides include sucrose, umbelliferose, lactose, planteose, isolychnose, α,α-trehalose, raffinose, lychnose, umbilicin, stachyose and verbascose.

Examples of polysaccharides include cellulose, quince seed, chondroitin sulfate, starch, galactan, dermatan sulfate, glycogen, gum Arabic, heparan sulfate, hyaluronic acid, gum tragacanth, keratan sulfate, chondroitin, xanthan gum, mucoitin sulfate, guar gum, dextran, keratosulfate, locust bean gum, succinoglycan and calonic acid.

Examples of amino acids include neutral amino acids (for example, threonine, cysteine, and the like) and basic amino acids (for example, hydroxylysine). In addition, examples of amino acid derivatives include sodium acyl sarcosinate (sodium lauroyl sarcosinate), acyl glutamates, sodium acyl β-alanine, glutathione and pyrrolidone carboxylic acid.

Examples of organic amines include monoethanolamine, diethanolamine, triethanolamine, morpholine, triisopropanolamine, 2-amino-2-methyl-1,3-propane diol and 2-amino-2-methyl-1-propanol.

Examples of pH-adjusting agents include buffering agents such as lactic acid-sodium lactate, citric acid-sodium citrate and succinic acid-sodium succinate.

Examples of vitamins include vitamins A, B1, B2, B6, C and E and derivatives thereof, pantothenic acid and derivatives thereof, and biotin.

Examples of antioxidants include tocopherols, dibutylhydroxytoluene, butylhydroxyanisole, and gallic acid esters.

Examples of other components able to be blended include preservatives (methylparaben, ethylparaben, butylparaben, phenoxyethanol, and the like); antiphlogistic agents (for example, glycyrrhizinic acid derivatives, glycyrrhetinic acid derivatives, salicylic acid derivatives, hinokitiol, zinc oxide, allantoin, and the like); whitening agents (for example, meadow saxifrage extracts, arbutin, and the like); a variety of extracts (for example, Phellodendron amurense, Coptis japonica, Lithospermum erythrorhizon, Chinese peony, Japanese green gentian, birch, sage, Eriobotrya japonica, carrot, aloe, common mallow, iris, grape, coix seed, sponge cucumber, lily, saffron, Cnidium officinale, ginger, Hypericum erectum, Restharrows, garlic, red pepper, Citrus reticulata peel, Angelica acutiloba, seaweed, and the like); activators (for example, royal jelly, photosensitizers, cholesterol derivatives, and the like); blood circulation promoters (for example, benzyl nicotinate, β-butoxyethyl nicotinate, capsaicin, zingerone, cantharidis tincture, ichthammol, tannic acid, α-borneol, tocopherol nicotinate, inositol hexanicotinate, cyclandelate, cinnarizine, tolazoline, acetylcholine, verapamil, cepharanthine, γ-oryzanol, and the like); antiseborrheic agents (for example, sulfur, thianthol, and the like); and anti-inflammatory agents (for example, tranexamic acid, thiotaurine, hypotaurine, and the like).

Products able to use the hair cosmetic composition of this invention are not particularly limited, but the hair cosmetic composition of this invention can be used in products requiring the advantageous effect of this invention, such as hair rinses, hair conditioners, hair treatments, hair packs, hair conditioning shampoos, brushing lotions, hair tonics, hair liquids, permanent wave agents, bleaches, hair dyes, hair manicure agents and hair coating agents.

In addition, the aqueous gelling agent represented by general formula (1) of component (A) of this invention, for which the viscosity of a 1 mass% aqueous solution at 25°C is 1,000 to 5,000 mPa·s, the clouding point of the 1 mass% aqueous solution containing is 60°C to 80°C, and the weight average molecular weight is 10,000 to 30,000, can be used in order to improve at least one, and preferably all, of the advantageous effects selected from among flow properties, temperature stability in terms of viscosity and texture of hair when applied thereto of a hair cosmetic composition containing either one of or both of a cationic surfactant and a cationic polymer.

### Examples

This invention will now be explained in detail by means of examples, but this invention is in no way limited to these examples, and may be altered as long as such alterations do not deviate from the scope of this invention. Moreover, in the examples etc. given below, % means mass percentage unless explicitly indicated otherwise.

First, component (A) used in the examples and comparative examples was produced.

### <Raw materials used to produce component (A)>

The raw materials used when producing component (A) are as follows.

Compound (2)-1: A compound in which R¹⁰ is a decyl group, R¹¹ is a dodecyl group, R¹² is an ethylene group and r = 20 in general formula (2)
Compound (2)-2: A compound in which R¹⁰ is an octyl group, R¹¹ is a decyl group, R¹² is an ethylene group and r = 20 in general formula (2)
Compound (2)-3: A compound in which R¹⁰ is a dodecyl group, R¹¹ is a tetradecyl group, R¹² is an ethylene group and r = 20 in general formula (2)
Compound (2)-4: A compound in which R¹⁰ is a decyl group, R¹¹ is a dodecyl group, R¹² is an ethylene group and r = 100 in general formula (2)
Compound (3)-1: A compound in which R¹³ is an ethylene group and t = 250 in general formula (3)
Compound (3)-2: A compound in which R¹³ is an ethylene group and t = 450 in general formula (3)
Compound (4)-1: Hexamethylene diisocyanate
Compound (4)-2: Dicyclohexylmethane diisocyanate (hydrogenated MDI)

Catalyst 1: Potassium laurate
Catalyst 2: Sodium laurate
Catalyst 3: Tetraisopropyl titanate
Catalyst 4: Dibutyltin dilaurate

### <Method for producing component (A)>

550 g (0.05 moles) of compound (3)-1 and 198 g (0.16 moles) of compound (2)-1 were placed in a four neck flask equipped with a temperature gauge, a nitrogen inlet tube and a stirrer, the temperature was increased to 40°C to 50°C, stirring was carried out until the components were homogeneously mixed, and once it had been confirmed that components were homogeneously mixed, 29.6 g (0.18 moles) of compound (4)-1 and 5.8 g (0.02 moles) of catalyst 1 were placed in the flask, and the system was purged with nitrogen. Component (A)-1 was then obtained by increasing the temperature to 80°C to 90°C and allowing a reaction to take place for 6 hours at this temperature.

Components (A)-2 to (A)-12 were produced by means of a similar method using the raw materials shown in Table 1. Moreover, components (A)-9 and (A)-10 were produced without using a catalyst, and components (A)-1 to (A)-12 were all produced using the same total mass of compound (2), compound (3) and compound (4), which are raw materials (that is, the mass of the overall reaction system).

**Table 1**

| | Raw materials | | | | Usage quantities (moles) of raw materials | | | |
|---|---|---|---|---|---|---|---|---|
| | Compound (2) | Compound (3) | Compound (4) | Catalyst | Compound (2) | Compound (3) | Compound (4) | Catalyst |
| Component (A)-1 | Compound (2)-1 | Compound (3)-1 | Compound (4)-1 | Catalyst 1 | 0.16 | 0.05 | 0.18 | 0.02 |
| Component (A)-2 | Compound (2)-1 | Compound (3)-1 | Compound (4)-1 | Catalyst 1 | 0.11 | 0.06 | 0.11 | 0.02 |
| Component (A)-3 | Compound (2)-2 | Compound (3)-1 | Compound (4)-1 | Catalyst 1 | 0.11 | 0.06 | 0.11 | 0.02 |
| Component (A)-4 | Compound (2)-3 | Compound (3)-1 | Compound (4)-1 | Catalyst 1 | 0.11 | 0.06 | 0.11 | 0.02 |
| Component (A)-5 | Compound (2)-4 | Compound (3)-1 | Compound (4)-1 | Catalyst 1 | 0.07 | 0.04 | 0.07 | 0.02 |
| Component (A)-6 | Compound (2)-1 | Compound (3)-2 | Compound (4)-1 | Catalyst 1 | 0.07 | 0.03 | 0.07 | 0.02 |
| Component (A)-7 | Compound (2)-1 | Compound (3)-1 | Compound (4)-2 | Catalyst 1 | 0.11 | 0.06 | 0.11 | 0.02 |
| Component (A)-8 | Compound (2)-1 | Compound (3)-1 | Compound (4)-1 | Catalyst 2 | 0.11 | 0.06 | 0.11 | 0.02 |
| Component (A)-9 | Compound (2)-1 | Compound (3)-1 | Compound (4)-1 | None | 0.16 | 0.05 | 0.18 | - |
| Component (A)-10 | Compound (2)-1 | Compound (3)-1 | Compound (4)-1 | None | 0.11 | 0.06 | 0.11 | - |
| Component (A)-11 | Compound (2)-1 | Compound (3)-1 | Compound (4)-1 | Catalyst 3 | 0.11 | 0.06 | 0.11 | 0.02 |
| Component (A)-12 | Compound (2)-1 | Compound (3)-1 | Compound (4)-1 | Catalyst 4 | 0.11 | 0.06 | 0.11 | 0.02 |

The constitutions and physical properties of obtained components (A)-1 to (A)-12 are shown in Table 2. Moreover, the obtained components (A) were mixtures of an aqueous gelling agent in which the value of g in general formula (1) is 1 to 10 and an aqueous gelling agent in which the value of g is 0, and the mass ratios of the aqueous gelling agents are also shown in Table 2.

### Method for measuring viscosity of 1 mass% aqueous solution

Measurement samples were prepared by adding water to the obtained components (A) so as to obtain a 1 mass% aqueous solution, and the viscosity of each aqueous solution was measured at 25°C using a B type viscometer (a TVB-10 available from Toki Sangyo Co., Ltd.).

### Method for measuring clouding point of 1 mass% aqueous solution

The thus prepared 1 mass% aqueous solution of component (A) was placed in a constant temperature bath, the temperature was gradually increased (at a rate of approximately 1°C/min), and the clouding point (°C) was taken to be the temperature at which turbidity occurred.

### Method for measuring weight average molecular weight

Weight average molecular weight was measured by means of gel permeation chromatography (GPC). Detailed measurement conditions are as follows.
GPC apparatus: HLC-8220GPC (Tosoh Corporation)
Column: Five columns connected in series, namely one TSKgel guard column SuperMP (HZ)-N column and four TSKgel SuperMultipore HZ-N columns.
Detector: RI
Sample concentration: 5 mg/ml (in THF solution)
Column temperature: 40°C
Standard sample: Polystyrene

In component (A), the mass ratio of a compound in which the value of g in general formula (1) is 0 and a compound in which the value of g is 1 to 10 is calculated from the area ratio of charts obtained from the GPC mentioned above.

**Table 2**

| | Constitution of component (A) | | Physical properties of 1% aqueous solution of component (A) | |
|---|---|---|---|---|
| | Weight average molecular weight | Mass ratio* | Viscosity (mPa·s, 25°C) | Clouding point (°C) |
| Component (A) - 1 | 16,000 | 85:15 | 4500 | 62 |
| Component (A) - 2 | 20,000 | 90:10 | 3500 | 65 |
| Component (A) - 3 | 18,000 | 90:10 | 1500 | 70 |
| Component (A) - 4 | 22,000 | 90:10 | 3500 | 65 |
| Component (A) - 5 | 25,000 | 90:10 | 3000 | 70 |
| Component (A) - 6 | 29,000 | 85:15 | 3000 | 60 |
| Component (A) - 7 | 20,000 | 90:10 | 3500 | 65 |
| Component (A) - 8 | 20,000 | 90:10 | 3500 | 65 |
| Component (A) - 9 | 12,000 | 80:20 | 500 | 90 |
| Component (A) - 10 | 15,000 | 98:2 | 800 | 85 |
| Component (A) - 11 | 13,000 | 85:15 | 5500 | 55 |
| Component (A) - 12 | 12,000 | 85:15 | 6000 | 53 |

| | | | | |
|---|---|---|---|---|
| *Mass ratio of (aqueous gelling agent in which value of g is 1 to 10) and (aqueous gelling agent in which value of g is 0) in general formula (1) | | | | |

### <Component B>

Component (B) used in the examples and comparative examples are shown below.
Component (B)-1: Cetyltrimethyl ammonium chloride
Component (B)-2: Behenyltrimethyl ammonium chloride
Component (B)-3: Dimethylaminopropylamide stearate
Component (B)-4: Diethylaminopropylamide stearate
Component (B)-5: Dimethylaminopropylamide behenate
Component (B)-6: Polyquaternium-6

### <Preparation of hair cosmetic compositions>

Hair cosmetic compositions were prepared using the above-mentioned components (A) and components (B) (see Tables 3 to 7). Moreover, the examples and comparative examples shown in Tables 3 to 7 are hair cosmetic compositions formulated as rinses.

**Table 3**

| | Example 1 | Example 2 | Example 3 | Example 4 | Example 5 | Example 6 | Example 7 | Example 8 | Comp. Example 1 | Comp. Example 2 | Comp. Example 3 | Comp. Example 4 | Comp. Example 5 |
|---|---|---|---|---|---|---|---|---|---|---|---|---|---|
| Component (A)-1 | 5.00 | | | | | | | | | | | | |
| Component (A)-2 | | 5.00 | | | | | | | | | | | |
| Component (A)-3 | | | 5.00 | | | | | | | | | | |
| Component (A)-4 | | | | 5.00 | | | | | | | | | |
| Component (A)-5 | | | | | 5.00 | | | | | | | | |
| Component (A)-6 | | | | | | 5.00 | | | | | | | |
| Component (A)-7 | | | | | | | 5.00 | | | | | | |
| Component (A)-8 | | | | | | | | 5.00 | | | | | |
| Component (A)-9 | | | | | | | | | 5.00 | | | | |
| Component (A)-10 | | | | | | | | | | 5.00 | | | |
| Component (A)-11 | | | | | | | | | | | 5.00 | | |
| Component (A)-12 | | | | | | | | | | | | 5.00 | |
| Component (B)-1 | 2.50 | 2.50 | 2.50 | 2.50 | 2.50 | 2.50 | 2.50 | 2.50 | 2.50 | 2.50 | 2.50 | 2.50 | 2.50 |
| Cetyl alcohol | 15.00 | 15.00 | 15.00 | 15.00 | 15.00 | 15.00 | 15.00 | 15.00 | 15.00 | 15.00 | 15.00 | 15.00 | 15.00 |
| L-glutamic acid | 0.50 | 0.50 | 0.50 | 0.50 | 0.50 | 0.50 | 0.50 | 0.50 | 0.50 | 0.50 | 0.50 | 0.50 | 0.50 |
| Purified water | Balance | Balance | Balance | Balance | Balance | Balance | Balance | Balance | Balance | Balance | Balance | Balance | Balance |
| Total | 500 | 500 | 500 | 500 | 500 | 500 | 500 | 500 | 500 | 500 | 500 | 500 | 500 |

| | | | | | | | | | | | | | |
|---|---|---|---|---|---|---|---|---|---|---|---|---|---|
| Unit: g | | | | | | | | | | | | | |

**Table 4**

| | Example 9 | Example 10 | Example 11 | Example 12 | Comp. Example 6 | Comp. Example 7 | Comp. Example 8 | Comp. Example 9 | Comp. Example 10 | Comp. Example 11 |
|---|---|---|---|---|---|---|---|---|---|---|
| Component (A)-1 | 5.00 | | | | | | | | | |
| Component (A)-2 | | 5.00 | | | | | | | | |
| Component (A)-3 | | | 5.00 | | | | | | | |
| Component (A)-4 | | | | 5.00 | | | | | | |
| Component (A)-9 | | | | | 5.00 | | | | | |
| Component (A)-10 | | | | | | 5.00 | | | | |
| Component (A)-12 | | | | | | | 5.00 | | | |
| Carboxyvinyl polymer | | | | | | | | 5.00 | | |
| Xanthan gum | | | | | | | | | 5.00 | |
| Component (B)-2 | 2.00 | 2.00 | 2.00 | 2.00 | 2.00 | 2.00 | 2.00 | 2.00 | 2.00 | 2.00 |
| Component (B)-3 | 0.50 | 0.50 | 0.50 | 0.50 | 0.50 | 0.50 | 0.50 | 0.50 | 0.50 | 0.50 |
| Stearyl alcohol | 15.00 | 15.00 | 15.00 | 15.00 | 15.00 | 15.00 | 15.00 | 15.00 | 15.00 | 15.00 |
| L-glutamic acid | 0.50 | 0.50 | 0.50 | 0.50 | 0.50 | 0.50 | 0.50 | 0.50 | 0.50 | 0.50 |
| Purified water | Balance | Balance | Balance | Balance | Balance | Balance | Balance | Balance | Balance | Balance |
| Total | 500 | 500 | 500 | 500 | 500 | 500 | 500 | 500 | 500 | 500 |

| | | | | | | | | | | |
|---|---|---|---|---|---|---|---|---|---|---|
| Unit: g * Carboxyvinyl polymer: Carbopol 980 (available from Nippon Lubrizol), Xanthan gum: Keltrol CG-7 (available from Sansho Co., Ltd.) | | | | | | | | | | |

**Table 5**

| | Example 13 | Example 14 | Comp. Example 12 | Comp. Example 13 | Comp. Example 14 |
|---|---|---|---|---|---|
| Component (A)-1 | 15.00 | | | | |
| Component (A)-2 | | 15.00 | | | |
| Component (A)-9 | | | 3.00 | | |
| Component (A)-10 | | | | 15.00 | |
| Component (B)-1 | 5.00 | 5.00 | 5.00 | 5.00 | 5.00 |
| Stearyl alcohol | 25.00 | 25.00 | 25.00 | 25.00 | 25.00 |
| L-glutamic acid | 1.00 | 1.00 | 1.00 | 1.00 | 1.00 |
| Purified water | Balance | Balance | Balance | Balance | Balance |
| Total | 500 | 500 | 500 | 500 | 500 |

| | | | | | |
|---|---|---|---|---|---|
| Unit: g | | | | | |

**Table 6**

| | Example 15 | Example 16 | Comp. Example 15 | Comp. Example 16 | Comp. Example 17 |
|---|---|---|---|---|---|
| Component (A)-1 | 5.00 | | | | |
| Component (A)-2 | | 5.00 | | | |
| Component (A)-9 | | | 5.00 | | |
| Component (A)-10 | | | | 5.00 | |
| Component (B)-3 | 1.00 | 1.00 | 1.00 | 1.00 | 1.00 |
| Component (B)-4 | 0.50 | 0.50 | 0.50 | 0.50 | 0.50 |
| Component (B)-5 | 0.50 | 0.50 | 0.50 | 0.50 | 0.50 |
| Cetyl alcohol | 1.00 | 1.00 | 1.00 | 1.00 | 1.00 |
| Stearyl alcohol | 2.50 | 2.50 | 2.50 | 2.50 | 2.50 |
| Behenyl alcohol | 2.50 | 2.50 | 2.50 | 2.50 | 2.50 |
| Purified water | Balance | Balance | Balance | Balance | Balance |
| Total | 500 | 500 | 500 | 500 | 500 |

| | | | | | |
|---|---|---|---|---|---|
| Unit: g | | | | | |

**Table 7**

| | Example 17 | Example 18 | Comp. Example 18 | Comp. Example 19 | Comp. Example 20 | Comp. Example 21 |
|---|---|---|---|---|---|---|
| Component (A)-2 | 5.00 | 5.00 | | | | |
| Component (A)-10 | | | 5.00 | 5.00 | | |
| Component (B)-1 | 15.00 | 10.00 | 15.00 | 10.00 | 15.00 | 10.00 |
| Component (B)-6 | | 20.00 | | 20.00 | | 20.00 |
| Dimethicone | 50.00 | | 50.00 | | 50.00 | |
| Stearyl alcohol | 25.00 | 25.00 | 25.00 | 25.00 | 25.00 | 25.00 |
| L-glutamic acid | 1.00 | 1.00 | 1.00 | 1.00 | 1.00 | 1.00 |
| Purified water | Balance | Balance | Balance | Balance | Balance | Balance |
| Total | 500 | 500 | 500 | 500 | 500 | 500 |

| | | | | | | |
|---|---|---|---|---|---|---|
| Unit: g | | | | | | |

### <Hair cosmetic performance evaluations>

The hair cosmetic compositions of Examples 1 to 18 and Comparative Examples 1 to 21, which were produced using the compositions shown in Tables 3 to 7, were evaluated in terms of (1) flow properties, (2) temperature stability in terms of viscosity and (3) hair texture when applied thereto. Evaluation criteria are as described below. Moreover, viscosity measurements were carried out using a B type viscometer (a TVB-10 available from Toki Sangyo Co., Ltd.).

### (1) Flow properties

In cases where a hair cosmetic composition was used as a rinse or conditioning shampoo, a sensory evaluation test was carried out as to whether or not the composition exhibited flow properties that facilitated such a use. By using a rinse or conditioning shampoo after hair has been washed with a shampoo, certain advantageous effects are required, such as coating the hair with a film, neutralizing hair that has become alkaline, suppressing evaporation of water, imparting gloss, moisture and softness, improving combability and preventing static electricity. Therefore, liquids having a low viscosity, such as water, have drawbacks such as being poor in terms of ease of application and spreading on the hair, being poor in terms of efficiency of covering hair with a film, and being difficult to handle. In addition, rinses and conditioning shampoos are often housed in pump type containers, and these hair cosmetics must have a viscosity that is suitable for being discharged from pump type containers. In addition, if the viscosity is too high also, drawbacks occur, such as being poor in terms of ease of application and spreading on the hair, being poor in terms of efficiency of covering hair with a film, and being difficult to handle. That is, in this test, the usefulness of the hair cosmetic composition when used as a rinse or conditioning shampoo and applied to hair was evaluated from the perspective of flow properties.

Specifically, 50 g of hair cosmetic compositions prepared using the compositions shown in Tables 3 to 7 were placed in 100 mL pump type containers and comprehensively evaluated by 10 people in terms of (i) ease of discharge from the pump type container and (ii) ease of application and spreading (ease of handling) when the hair cosmetic was discharged into the palm of the hand and applied to hair, with the highest score being 5 points (and the lowest score being 1 point), and the total scores were evaluated as A to E using the criteria below. In these evaluations, B or higher was taken to be a pass.

A: Total score of 45 to 50 points
B: Total score of 40 to 44 points
C: Total score of 35 to 39 points
D: Total score of 30 to 34 points
E: Total score of 29 points or less

### (2) Temperature stability in terms of viscosity

100 g of the samples shown in Tables 3 to 7 were placed in transparent glass containers and stored for 2 months in constant temperature baths at temperatures of 25°C or 50°C. The state (viscosity) of each sample was then measured at 25°C, and the difference between the viscosity of a sample stored at 25°C (standard value) and the viscosity of a sample stored at 50°C was calculated as the degree of change in viscosity from these results, and evaluated as A to D, as shown below. In these evaluations, B or higher was taken to be a pass.

A: The degree of change in the viscosity of a sample stored at 50°C compared to that of a sample stored at 25°C was not more than 10%.
B: The degree of change in the viscosity of a sample stored at 50°C compared to that of a sample stored at 25°C was more than 10% but not more than 20%.
C: The degree of change in the viscosity of a sample stored at 50°C compared to that of a sample stored at 25°C was more than 20% but not more than 30%.
D: The degree of change in the viscosity of a sample stored at 50°C compared to that of a sample stored at 25°C was more than 30%.

### (3) Texture of hair when applied thereto

Specifically, texture of hair when applied thereto means a comprehensive evaluation of (I) the softness of hair immediately after washing the hair with shampoo, applying samples having the compositions shown in Tables 3 to 7 and then rinsing out the samples, (II) a feeling of moisture in the hair after completely drying the hair with a hair dryer, and (III) combability (smoothness). The texture mentioned above was comprehensively evaluated by 10 people for the samples shown in Tables 3 to 7, with the highest score being 5 points (and the lowest score being 1 point), and the total scores were evaluated as A to E using the criteria below. In these evaluations, B or higher was taken to be a pass.

A: Total score of 45 to 50 points
B: Total score of 40 to 44 points
C: Total score of 35 to 39 points
D: Total score of 30 to 34 points
E: Total score of 29 points or less

The evaluation results are shown below.

**Table 8**

| | Example 1 | Example 2 | Example 3 | Example 4 | Example 5 | Example 6 | Example 7 | Example 8 | Comp. Example 1 | Comp. Example 2 | Comp. Example 3 | Comp. Example 4 | Comp. Example 5 |
|---|---|---|---|---|---|---|---|---|---|---|---|---|---|
| Flow properties | A | A | B | B | A | B | B | A | C | C | C | C | E |
| Temperature stability in terms of viscosity | A | A | A | A | A | A | A | A | B | B | C | C | D |
| Hair texture when applied thereto | A | A | A | A | A | A | A | A | C | C | C | C | E |

**Table 9**

| | Example 9 | Example 10 | Example 11 | Example 12 | Comp. Example 6 | Comp. Example 7 | Comp. Example 8 | Comp. Example 9 | Comp. Example 10 | Comp. Example 11 |
|---|---|---|---|---|---|---|---|---|---|---|
| Flow properties | A | A | B | B | C | C | C | E | C | E |
| Temperature stability in terms of viscosity | A | A | A | A | B | B | C | D | C | D |
| Hair texture when applied thereto | A | A | A | A | C | C | C | D | C | E |

**Table 10**

| | Example 13 | Example 14 | Comp. Example 12 | Comp. Example 13 | Comp. Example 14 |
|---|---|---|---|---|---|
| Flow properties | A | A | C | C | E |
| Temperature stability in terms of viscosity | A | A | B | B | D |
| Hair texture when applied thereto | A | A | C | C | E |

**Table 11**

| | Example 15 | Example 16 | Comp. Example 15 | Comp. Example 16 | Comp. Example 17 |
|---|---|---|---|---|---|
| Flow properties | A | A | B | B | E |
| Temperature stability in terms of viscosity | A | A | B | B | D |
| Hair texture when applied thereto | A | A | C | C | E |

**Table 12**

| | Example 17 | Example 18 | Comp. Example 18 | Comp. Example 19 | Comp. Example 20 | Comp. Example 21 |
|---|---|---|---|---|---|---|
| Flow properties | A | A | C | C | E | E |
| Temperature stability in terms of viscosity | A | A | B | B | D | D |
| Hair texture when applied thereto | A | A | B | B | C | C |

It was understood from the results that the product of this invention is a hair cosmetic composition favorable in terms of (1) flow properties, (2) temperature stability in terms of viscosity and (3) texture of hair when applied thereto.

### Industrial Applicability

The hair cosmetic composition of this invention is a hair cosmetic composition that exhibits flow properties suitable for a hair cosmetic, has high temperature stability in terms of viscosity and imparts a more favorable texture to hair when applied thereto compared to conventional hair cosmetics. From a user's point of view, this hair cosmetic composition exhibits excellent feeling of use, meets high expectations and is extremely useful.

## Claims

1. A hair cosmetic composition comprising components (A) and (B) below, wherein the component (A) presents a viscosity of an aqueous solution containing 1 mass% of at 25°C of 1,000 to 5,000 mPa·s and a clouding point of the aqueous solution containing 1 mass% of component (A) of 60°C to 80°C, and has a weight average molecular weight of 10,000 to 30,000; component (A): an aqueous gelling agent represented by general formula (1) below: 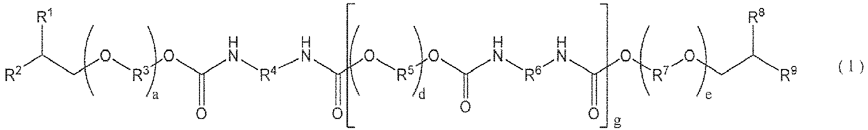 wherein R¹, R², R⁸ and R⁹ each independently denote a hydrocarbon group having 4 to 20 carbon atoms, R³, R⁵ and R⁷ each independently denote a divalent hydrocarbon group having 2 to 4 carbon atoms, R⁴ and R⁶ each independently denote a divalent hydrocarbon group having 3 to 16 carbon atoms, a and e each independently denote a number from 10 to 100, d denotes a number from 100 to 500, and g denotes a number from 0 to 10; and
component (B): either one of or both of a cationic surfactant and a cationic polymer

2. The hair cosmetic composition according to claim 1, wherein R¹, R², R⁸ and R⁹ in the aqueous gelling agent represented by general formula (1) as the component (A) each independently denote an aliphatic hydrocarbon group having 10 to 12 carbon atoms.

3. The hair cosmetic composition according to claim 1 or 2, wherein R⁴ and R⁶ in the aqueous gelling agent represented by general formula (1) as the component (A) each independently denote a divalent aliphatic hydrocarbon group having 4 to 8 carbon atoms.

4. The hair cosmetic composition according to any one of claims 1 to 3, wherein the component (B) is either one of or both of an alkyltrimethyl ammonium salt represented by general formula (5) and an amide compound represented by general formula (11):
R¹⁴-N⁺(CH₃)₃X⁻ (5)
wherein R¹⁴ denotes an alkyl group having 10 to 24 carbon atoms, and X- denotes a halide ion, a methosulfate ion, an ethosulfate ion, a methophosphate ion, an ethophosphate ion or a methocarbonate ion;
R²²-CONH-(R²³)-N-(R²⁴)₂ (11)
wherein R²² denotes an alkyl group having 10 to 24 carbon atoms, R²³ denotes an alkylene group having 2 to 4 carbon atoms, and the R²⁴ moieties each independently denote an alkyl group having 1 to 4 carbon atoms.

5. The hair cosmetic composition according to any one of claims 1 to 3, wherein component (B) is a mixture of a cationic surfactant and a cationic polymer.

6. A method for producing an aqueous gelling agent represented by general formula (1) below and used to produce a hair cosmetic composition, the method comprising: adding an alcohol compound represented by general formula (2) at a molar ratio of 1.5 to 2.4 and a polyalkylene glycol represented by general formula (3) at a molar ratio of 0.5 to 1.4 to a diisocyanate compound represented by general formula (4) at a molar ratio of 2, and allowing these components to react in the presence of a higher fatty acid metal salt; 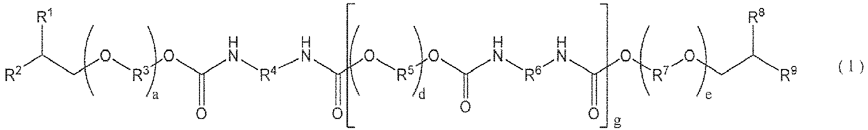 wherein R¹, R², R⁸ and R⁹ each independently denote a hydrocarbon group having 4 to 20 carbon atoms, R³, R⁵ and R⁷ each denote represent a divalent hydrocarbon group having 2 to 4 carbon atoms, R⁴ and R⁶ each independently denote a divalent hydrocarbon group having 3 to 16 carbon atoms, a and e each independently denote a number from 10 to 100, d denotes a number from 100 to 500, and g denotes a number from 0 to 10; 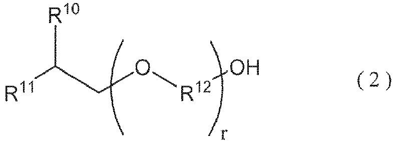 wherein R¹⁰ and R¹¹ each independently denote a hydrocarbon group having 4 to 20 carbon atoms; R¹² denotes a divalent hydrocarbon group having 2 to 4 carbon atoms; and r denotes a number from 10 to 100; 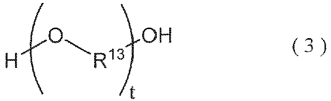 wherein R¹³ denotes a divalent hydrocarbon group having 2 to 4 carbon atoms, and t denotes a number from 100 to 500; and
OCN-Q-NCO (4)
wherein Q denotes a divalent hydrocarbon group having 3 to 16 carbon atoms.

7. The production method according to claim 6, wherein the higher fatty acid metal salt is one or more kinds of higher fatty acid metal salts selected from the group consisting of lauric acid metal salts, myristic acid metal salts, palmitic acid metal salts, stearic acid metal salts, and oleic acid metal salts.

8. The production method according to claim 7, wherein the higher fatty acid metal salt is a lauric acid metal salt.

9. A method for producing a hair cosmetic composition, the method comprising:
a step of obtaining an aqueous gelling agent represented by general formula (1) below by adding an alcohol compound represented by general formula (2) at a molar ratio of 1.5 to 2.4 and a polyalkylene glycol represented by general formula (3) at a molar ratio of 0.5 to 1.4 to a diisocyanate compound represented by general formula (4) at a molar ratio of 2, and allowing these components to react in the presence of a higher fatty acid metal salt, and
a step of combining said aqueous gelling agent with either one of or both of a cationic surfactant and a cationic polymer; 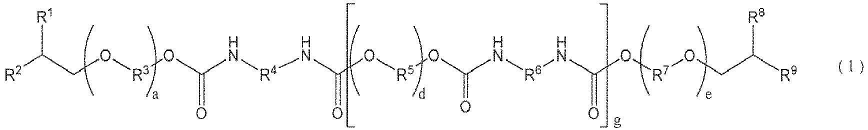 wherein R¹, R², R⁸ and R⁹ each independently denote a hydrocarbon group having 4 to 20 carbon atoms, R³, R⁵ and R⁷ each denote represent a divalent hydrocarbon group having 2 to 4 carbon atoms, R⁴ and R⁶ each independently denote a divalent hydrocarbon group having 3 to 16 carbon atoms, a and e each independently denote a number from 10 to 100, d denotes a number from 100 to 500, and g denotes a number from 0 to 10; 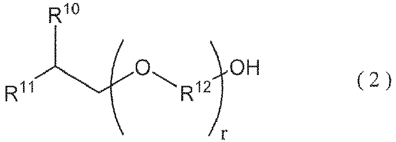 wherein R¹⁰ and R¹¹ each independently denote a hydrocarbon group having 4 to 20 carbon atoms, R¹² denotes a divalent hydrocarbon group having 2 to 4 carbon atoms, and r denotes a number from 10 to 100; 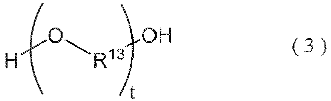 wherein R¹³ denotes a divalent hydrocarbon group having 2 to 4 carbon atoms, and t denotes a number from 100 to 500; and
OCN-Q-NCO (4)
wherein Q denotes a divalent hydrocarbon group having 3 to 16 carbon atoms.
